# EUROPEAN PATENT SPECIFICATION

(11) **EP 0 399 653 B1**
(45) Date of publication and mention of the grant of the patent: **19.10.1994**
(21) Application number: 90304271.1
(22) Date of filing: 20.04.1990
(51) Int. Cl.: C07D 471/04, A61K 31/435

(54) **Anticonvulsant triazolo[4,5-c]pyridine derivatives**
Triazolo[4,5-c]pyridinderivate mit krampflindernder Wirkung
Dérivés de la triazolo[4,5-c]pyridine doués d'une activité anticonvulsive

(30) Priority: 21.04.1989 GB 8909136
(43) Date of publication of application: 28.11.1990
(73) Proprietor: THE WELLCOME FOUNDATION LIMITED, London NW1 2BP (GB)
(72) Inventor: Kelley, James Leroy, Raleigh, North Carolina 27614 (US)
(74) Representative: Stott, Michael John

(56) References cited:
- EP-A- 0 288 431
- DE-A- 2 521 920

## Description

The present invention relates to a class of pyridine compounds, particularly triazolopyridines, to pharmaceutical compositions containing them, and to methods for their preparation, to their use in medicine and to methods of treating central nervous system disorders, such as epilepsy in mammals.

European patent application no. 85302321.6, published under no. 157637 discloses a class of 6-amino-9-(fluorobenzyl)-9H-purines as having anticonvulsant activity. However, these compounds, when administered to man caused emesis and nausea and therefore are unsuitable for use as an antiepileptic drug. Analogues of these compounds, notably 3-deazapurines have been reported (J.Heterocyclic Chem, 25, 1255 (1988)) to have anticonvulsant activities but were found to be more toxic.

European patent application no. 88810212.6, published under no. 0288431 after the priority date hereof, discloses a class 3H-1,2,3-Triazolo[4,5-d] pyrimidines as putative anticonvulsant agents.

Epilepsy is a collective designation for a group of chronic central nervous system disorders having in common the occurrence of sudden and transitory episodes (seizure) of abnormal phenomena of motor (convulsions), sensory, autonomic or psychic origin. The seizures are nearly always correlated with abnormal electrical activity of the brain.

The incidence of epilepsy is estimated to be approximately 1% of the total world population. A fairly large proportion (10-20%) is not adequately controlled by currently available therapies; such patients are described as refractory. Those drugs which are currently available to the medical practitioner suffer from severe limitation in use and also have a number of side effects. It is therefore clearly apparent that there is a need for new antiepileptic drugs.

The present inventors have surprisingly found that a series of novel triazolopyridines have potent anticonvulsant activity.

Accordingly, in a first aspect of the present invention there is provided compound of formula I 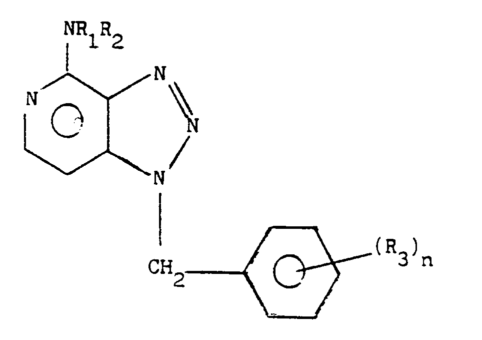
or a salt thereof.
where R₁ and R₂ may be the same or different and are selected from hydrogen, C₁₋₄ straight or C₃₋₄ branched alkyl or cyclo C₃₋₄ alkyl or cyclopropylmethyl;
and R₃ is hydrogen or halo
and n is either 1 or 2.

Particularly preferred compounds and salts thereof are those where R₃ is fluoro; more particularly when R₃ is selected from 2-fluoro; 3-fluoro; 4-fluoro; 2,6-difluoro; 2,5-difluoro; 2,4-difluoro; 2,3-difluoro; 3,4-difluoro and 3,5-difluoro.

The following compounds and their salts are particularly preferred:
1-(2-Fluorobenzyl)-4-(methylamino)-1H-1,2,3-triazolo[4,5-c]pyridine
4-(cyclopropylamino)-1-(2-fluorobenzyl)-1H-1,2,3-triazolo[4,5-c]pyridine
4-(ethylamino)-1-(2-fluorobenzyl)-1H-1,2,3-triazolo[4,5-c]pyridine
4-(dimethylamino)-1-(2-fluorobenzyl)-1H-1,2,3-triazolo[4,5-c]pyridine
1-(2,6-difluorobenzyl)-4-(methylamino)-1H-1,2,3-triazolo[4,5-c] pyridine
4-amino-1-(2,6-difluorobenzyl)-1H-1,2,3-triazolo[4,5-c]pyridine
1-(2,5-difluorobenzyl)-4-(methylamino)-1H-1,2,3-triazolo[4,5-c] pyridine
1-benzyl-4-(methylamino)-1H-1,2,3-triazolo[4,5-c]pyridine
Two compounds, namely, 1-(2,6-Difluoro-benzyl)-4-(methylamino)-1H-1,2,3-triazolo[4,5-c]pyridine and 4-Amino-1-(2,6-difluorobenzyl)-1H-1,2,3-triazolo[4,5-c]pyridine are particularly preferred being potent anticonvulsant and non-emetic agents.

Suitable acid addition salts of the compounds of formula I include those formed with both organic and inorganic acids. Such acid addition salts will normally be pharmaceutically acceptable.

Thus, preferred salts include those formed from hydrohalic, eg. hydrochloric, sulphuric, citric, isethionic, tartaric, phosphoric, lactic, pyruvic, acetic, succinic, oxalic, fumaric, maleic, lactobionic, oxaloacetic, methanesulphonic, p-toluenesulphonic and benzenesulphonic acids.

There is also provided the first medical use of the novel compounds of the present invention or pharmaceutically acceptable salts thereof, as hereinbefore defined. Preferably this will be for the treatment of CNS disorders, and in particular epilepsy, in humans. The compounds of the present invention have phenytoin like activity and are particularly useful in the treatment of primary generalised tonic-clonic (grand mal) epilepsy.

In a further aspect, there are provided pharmaceutical formulations comprising a compound of the present invention in admixture with a pharmaceutically acceptable carrier.

The pharmaceutically acceptable carriers present in the compositions of this invention are materials recommended for the purpose of administering the medicament. These may be liquid or solid materials, which are otherwise inert or medically acceptable and are compatible with the active ingredients.

These pharmaceutical compositions may advantageously be given orally, but may also be given parenterally, used as a suppository, or applied topically as an ointment, cream or powder. Oral and parenteral administration of the compositions are preferred.

For oral administration, fine powders or granules will contain diluting, dispersing and/or surface active agents, and may be presented in a draught, in water or in a syrup, in capsules or sachets in the dry state or in non-aqueous suspension wherein suspending agents may be included, or in a suspension in water or syrup. Where desirable or necessary, flavouring, preserving, suspending, thickening or emulsifying agents can be included.

For parenteral administration, the compounds may be presented in sterile aqueous injection solutions which may contain anti-oxidants or buffers.

As stated above, the free base or a salt thereof may be administered in its pure form unassociated with other derivatives, in which case a capsule or sachet is the preferred carrier.

Alternatively the active compound may be presented in a pure form as an effective unit dosage, for instance compressed as a tablet or the like.

Other compounds which may be included are, for example, medically inert ingredients, eg. solid and liquid diluents such as lactose, starch or calcium phosphate for tablet or capsule; olive oil or ethyl oleate for soft capsules; and water or vegetable oil for suspensions or emulsions; lubricating agents such as talc or magnesium stearate; gelling agents such as colloidal clays; thickening agents such as gum tragacanth or sodium alginate; and other therapeutically acceptable accessory ingredients such as humectants, preservatives, buffers, and antioxidants which are useful as carriers in such formulations.

Tablets or other forms of presentation provided in discrete units may conveniently contain an amount of a novel compound as hereinbefore defined which is effective at such dosage or a multiple of the same, for instance, units containing 5mg to 500mg, usually around 10mg to 250mg.

The pharmaceutical compositions of the present invention will be prepared by the admixture of a novel compound or salt as hereinbefore defined with a pharmaceutically acceptable carrier. Conventional pharmaceutical excipients may be admixed as required.

As indicated above, the compounds of the formula I are generally useful in treating such disorders by administration to the human or animal recipient by a route selected from oral, parenteral (including subcutaneous, intradermal, intramuscular and intravenous) and rectal. The size of an effective dose of a compound will depend upon a number of factors including the mammal under treatment (for example cat, dog or human), the type of epilepsy involved for example grand mal, focal seizures and psychomotor convulsions, the severity of the condition to be treated and the route of administration, and will ultimately be at the discretion of the attendant physician. In guiding him in assessing the efficacy and acceptability of a regimen the physician will have recourse to changes in the recipient's gross condition as treatment progresses.

Such an effective dose will generally be in the range 0.3 to 15 mg/kg bodyweight of animal or human recipient given three times per day, preferably in the range 0.5 to 7mg/kg bodyweight and most preferably in the range of 1 to 2mg/kg bodyweight. For the average human of 70kg bodyweight at 1.0 mg/kg the dose would be 70mg. Unless otherwise indicated all weights are calculated as the hydrochloride of formula I. For other salts the figures would be amended proportionately. The desired dose may be preferably presented as between two and four sub-doses administered at appropriate intervals throughout the day.

The present invention also provides a process for the preparation of a compound of formula I, which process comprises the reaction of an amine HNR₁R₂ with a compound of formula II 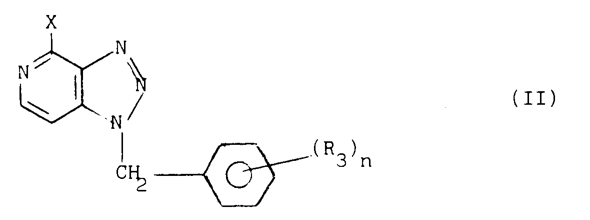
where R₁, R₂ and R₃ and n are as hereinbefore defined and X is a leaving group.

Compounds of formula II can be prepared by reacting the appropriate diaminopyridines of formula III 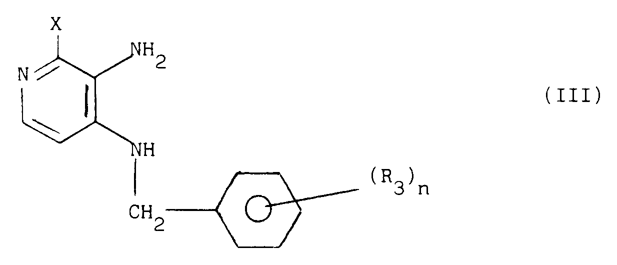
where X and R₃ and n are as hereinbefore defined with sodium nitrite in hydrohalic acid, particularly hydrochloric acid.

Compounds of formula III can be prepared by reductive halogenation of a compound of formula IV 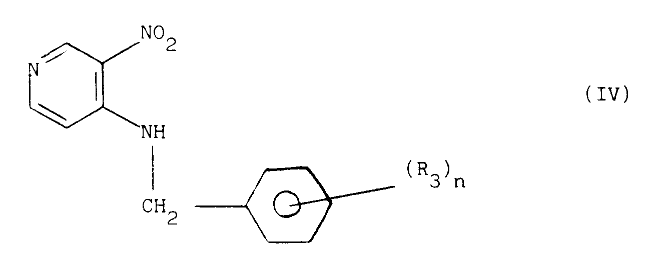
wherein R₃ and n are as hereinbefore defined by treating, for example, with stannous chloride in hot concentrated hydrochloric acid.

Compounds of formula IV can be obtained by reacting compounds of formula V, wherein R⁴ is chloro or OR where R is C₁₋₆alkyl, 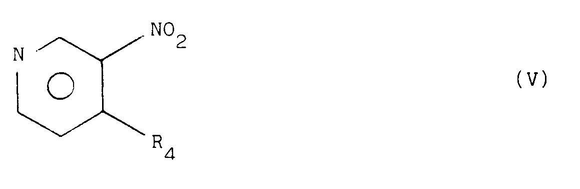
with the appropriate benzyl amine.

Compounds of formula V can be prepared from 4-hydroxypyridine by nitrating with fuming nitric acid and reacting the resulting 4-hydroxy-3-nitropyridine with phosphorous pentachloride, followed by reaction with an appropriate alcohol.

The reaction of a compound of formula II with a mono or disubstituted amine will take place in any suitable solvent, preferably this will be a polar solvent such as a C₁₋₄ alkanol, water or acetonitrile. Where appropriate the amine may be used as co-solvent. The reaction will be carried out at non-extreme temperatures eg. 0-180°C suitably at 15-120°C and conveniently at room temperature.

Suitable leaving groups X include halogen, C₁₋₆ alkylthio, C₆₋₁₀ arylthio, C₇₋₁₂ aralkylthio or C₁₋₄ alkyl, phenyl, benzyl, phenylethyl or naphthylmethyl, substituted sulphonyl or sulphinyl. Preferred leaving groups include halogen, particularly chlorine.

Alternatively compounds of formula II can be prepared by the reaction of a compound formula VI with a compound of formula VII (Aldrich) 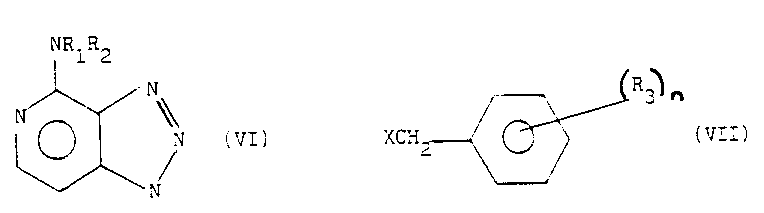
wherein R₁, R₂, R₃, n and X are hereinbefore defined.

The triazolo [4,5-d] pyridines of formula (VI) can be prepared in a manner analogous to the preparation of formula (II) by reacting a compound of 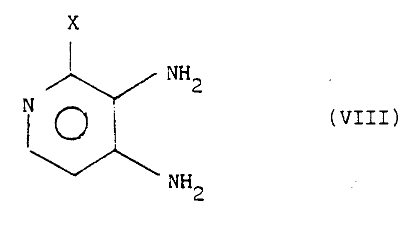
with sodium nitrite in hydrohalic acid and corresponding amination, with suitable aminating agents.

The following examples serve to illustrate the present invention.

### Example 1

### Preparation of 1-(2-fluorobenzyl)-4-(methylamino)-1H-1,2,3-triazolo[4,5-c]pyridine.

A) Preparation of 4-[(2-fluorobenzyl)amino]-3-nitropyridine
   A mixture of 4-chloro-3-nitropyridine (22.19g, 0.140mol), 2-fluoro-benzylamine (Aldrich) (16.04g, 0.128mol) and water/dioxane (8:3) (220ml) was stirred at ambient temperature for 30 min. The reaction mixture was cooled in an ice-bath and triethylamine (107ml) was added dropwise. A precipitate formed, and the mixture was stirred for 30 min at ice bath temperature and at ambient temperature for 15 hr. The suspension was diluted with water (300ml). The solids were collected, dried, dissolved in dichloromethane and added to Silica Gel 60. This mixture was spin evaporated in vacuo, and the residual solids were added to a column (5cm x 25cm) of Silica Gel 60 wetted with dichloromethane/ethyl acetate (5:1). The column was eluted with the same solvent using the flash chromatography technique. The appropriate fractions were combined and spin evaporated in vacuo to give 25.5g (80%) of 4-[(2-fluorobenzyl)amino]-3-nitropyridine as a bright yellow solid, mp.= 108-109°; UV (0.1 N hydrochloric acid + 5% methanol) λmax 231mm (ξ16600), 268 nm (ξ14700) , 345 nm (ξ4200); pH 7.0 buffer + 5% methanol) λmax 237 nm (ξ22600), 378 nm (ξ5900); (0.1 N sodium hydroxide + 5% methanol) λmax 237 nm (ξ20500), 378 nm (ξ5400); 1H nmr (DMSO-d₆): δ 9.05 (s, 1H, pyridine H-2), 8.89 (t, 1H,NH), 8.22 (d,1H,J=6 Hz, pyridine H-6), 7.4-7.1 (m, 4H,Ar), 6.84 (d,1H,J=6 Hz, pyridine H-5), 4.71 (d,2H,J=6 Hz, CH₂).
B. Preparation of 3-amino-2-chloro-4-[(2-fluorobenzyl)amino]pyridine
   A mechanically stirred solution of 4-[(2-fluorobenzyl)amino]-3-nitropyridine (12.31g, 49.8mmol) in concentrated hydrochloric acid (118ml) was heated to 90° under a nitrogen atm. Stannous chloride dihydrate (55.56g, 246mmol) was added in small portions over a 5 min period (the oil bath was removed until the reaction subsided). After an additional 30 min at 90°, the reaction mixture was cooled, diluted with water (200ml) and spin evaporated in vacuo. The residue was diluted with water (200ml) and cooled in an ice-bath, while concentrated ammonium hydroxide was added to adjust the pH to 7-8. The solids were collected and allowed to air dry overnight. The solid was treated with ethyl acetate and filtered (12x200ml), the combined extracts were washed with water, dried (sodium sulphate) and spin evaporated in vacuo. The residue was combined with the product from a separate reaction (12g, 48.5mmoles) and dissolved in ethyl acetate. This solution was added to Silica Gel 60 and spin evaporated in vacuo. The residual solids were introduced to a column (5cm x 30cm) of Silica Gel 60 wetted with ethyl acetate. The column was eluted with ethyl acetate using flash chromatography. The appropriate fractions were combined and spin evaporated in vacuo to give 20.8g (84%) of 3-amino -2- chloro-4-[2-fluorobenzyl)amino]pyridine as a white solid, mp.= 185-187°; UV (0.1 M hydrochloric acid + 5% methanol), λmax 233 nm (ξ17900), 302 nm (ξ14900); (pH 7.0 buffer + 5% methanol), λmax 262 nm (ξ11200), (0.1 N sodium hydroxide + 5% methanol), λmax 262 nm (ξ10700), 1H nmr (DMSO-d₆): δ 7.36-7.15 (complex m, 5H,Ar + pyridine H-6), 6.35-6.30 (d,1H,pyridine H-5), 6.27-6.33 (overlapping,1H,NH), 4.84 (2,2H,NH₂), 4.41 (d,2H,J=5 Hz, CH₂Ar).
   B(i) Alternative preparation of 3-amino-2-chloro-4-[(2-fluorobenzyl)amino] pyridine
      A mechanically stirred solution of 4-[(2-fluorobenzyl)amino]3-nitro pyridine (37.62g, 152.17 mmol) in concentrated hydrochloric acid (400mL) was heated to 90° under a nitrogen atmosphere. Stannous chloride dihydrate (174.88g, 775.07mmol) was added in small portions over a 20 min period. When the reaction became vigorous, the oil bath was removed until the reaction subsided. After 45 min at an increased temperature to 120° for 45 min, the reaction mixture was cooled, diluted with water (350mL) and spin evaporated in vacuo. The residue was diluted with water (600mL), cooled in an ice-bath and concentrated ammonium hydroxide (250mL) was added to adjust the pH to 7-8. The solid were collected and allowed to air dry overnight. The solid cake was broken up and suspended in ethyl acetate (3 x 1L). Each suspension was refluxed with the refluxing time varying from 3.5 hrs to 18 hrs. The ethyl acetate was filtered and evaporated in vacuo to yield 3-amino-2-chloro-4-[(2-fluorobenzyl)amino]pyridine (27.13g, 71%) as an off-white solid, mp.=181-187°. The analytical sample was prepared by essentially the same preparative method and was purified by column chromatography.
C Preparation of 1-(2-fluorobenzyl)-4-(methylamino)-1H-1,2,3-triazolo [4,5-c]pyridine hydrochloride
   To an ice cold solution of 3-amino-2-chloro-4-[(2-fluorobenzyl)amino] pyridine (4.0g, 15.9mmol), 1N hydrochloric acid (40ml), concentrated hydrochloric acid (15ml) and ethanol (120ml) was added sodium nitrite (1.31g, 18.9mmol). The solution was stirred for 15 min and 40% aqueous methylamine (100ml) was added. The solution was refluxed with stirring for 30 min. The reaction was cooled, and the solid was collected and washed with water. The slid was dissolved in hot ethanol (155ml) and then diluted with concentrated hydrochloric acid (55ml). The solution was cooled and the white solid was collected to give 3.72g (79%) of 1-(2-fluorobenzyl)-4-(methylamino)-1H -1,2,3-triazolo[4,5-c]pyridine hydrochloride, mp=280-282°C (dec); UV (0.1 N hydrochloric acid + 5% methanol) λmax 280 nm (ξ12900); (pH 7.0 buffer + 5% methanol) λmax 302 nm (ξ8600); (0.1 N sodium hydroxide + 5% methanol) λmax 306 nm (ξ9000); 1H nmr (DMSO-d₆): δ 10.2 (br m, 1H, NH), 7.84 (d, 1H, J=7 Hz, pyridine H-6), 7.5-7.2 (m, 5H, Ar + pyridine H-7), 6.03 (s, 2H, Ch₂), 3.13 (br, s, 3H, CH₃).

### Example 2

### Preparation of 4-(cyclopropylamino)-1-(2-fluorobenzyl)-1H-1,2,3-triazolo [4,5-c] pyridine

This compound was prepared in an analogous manner to that of example 1 with the replacement of methylamine in example 1C with cyclopropylamine. It was precipitated from free base in EtOH with concentrated HCl. Yield 72%, mp= 245-247°C. UV: (0.1 N HCl) λmax 283 (ξ13800); (pH 7 buffer) λmax 300 (ξ9400); (0.1 N NaOH) λmax 306 (ξ10000).

### Example 3

### Preparation of 4-(ethylamino)-1-(2-fluorobenzyl)-1H-1,2,3-triazolo[4,5-c] pyridine

This compound was prepared in an analogous manner to that of example 1 with the replacement of methylamine in example 1C with ethylamine. The compound was precipitated from a solution of the free base in EtOH with concentrated HCl. Yield 84% mp. 258-260 UV: (0.1 N HCl) λmax 282.8 nm (ξ13400); (pH 7 buffer) λmax 306.8 (ξ9400); (0.1 N NaOH) λmax 308.8 (ξ9700).

### Example 4

### Preparation of 4-(dimethylamino)-1-(2-fluorobenzyl)-1H-1,2,3-triazolo [4,5-c]pyridine

This compound was prepared in an analogous manner to the compound in example 1 with the replacement of methylamine in example 1C with dimethylamine. The compound was recrystallised from propan-2-ol. Yield 69%, mp.= 248-252° UV:(0.1 N HCl) λmax 289.6 (ξ15200); (pH 7 buffer) λmax 318.0 (ξ10600); (0.1 N NaOH) λmax 318.0 (ξ11900).

### Example 5

### Preparation of 1-(2,6-difluorobenzyl)-4-(methylamino)-1H-1,2,3-triazolo [4,5-c] pyridine

A Preparation of 4-[(2,6-difluorobenzyl)amino]-3-nitropyridine
   This compound was prepared in a manner analogous to example 1(A) but replacing 2-fluorobenzylamine with 2,6-difluorobenzylamine, (Aldrich).
B Preparation of 3-Amino-2-chloro-4[(2,6-difluorobenzyl)amino]pyridine
   Procedure: A 3-neck 5-L flask, equipped with a thermometer, reflux condenser, nitrogen inlet and a mechanical stirrer was charged with 4-[(2,6-difluorobenzyl)amino]3-nitropyridine and concentrated hydrochloric acid. The solution was heated on a steam bath to 90°C and stannous chloride dihydrate was added portionwise.¹ After heating the suspension at 90°C for 1.5 hours, the condenser was replaced with a distillation apparatus and approximately 1.5L of H₂O removed by distillation at 100mm Hg.² The remaining suspension was cooled in an ice bath and neutralised to pH 7 with concentrated ammonium hydroxide. The pasty solid was collected by filtration and dried overnight on a Buechner funnel. The still damp solid was transferred to a 12-L flask equipped with a mechanical stirrer and reflux condenser and charged with ethyl acetate. With rapid stirring, the suspension was heated at reflux for 3.5 hours on a steam bath, cooled slightly, filtered using positive nitrogen pressure, and the filtrate concentrated in vacuo. The resulting oily solid was slurried in ethyl ether, solids collected by filtration and dried overnight on a Buechner funnel to yield 123g (83.1%) of 3-amino-2-chloro-4-[(2,6-difluorobenzyl)amino]pyridine.
   TLC (silica gel, EtOAc/ether, 1:1) R_{f}=0.71;
   mp = 221-223°C; 1H NMR (DMSO-d₆) δ,J=5.1 Hz, 2H, CH₂), 4.84 (s, 2H, NH₂), 6.06(t, J=5.1, Hz, 1H, NH), 6.54(d,J=5.4Hz, 1H, C-5H), 7.01-7.65 (m, 3H, C-3'H, C-4'H, C-5'H), 7.44 (d, J=5.3 Hz, 1H, C-6H); Elemental analysis calculated C, 53.45; H, 3.74; N, 15.58; Cl, 13.15; F, 14.08; Found: C, 53.60; H, 3.69; N, 15.51; Cl, 13.09; F, 14.12.
   1. Copious gas evolution occurred upon the addition of SnCl₂-2H₂0.
   2. The H₂O was carefully distilled off under house vacuum. Bumping occurred several times.
C Preparation of 1-(2,6-Difluorobenzyl)-4-(methylamino)-1H-1,2,3-triazolo[4,5-c]pyridine Hydrochloride
   Procedure: A 5-L flask equipped with mecahnical stirrer, condenser and thermometer was charged with concentrated hydrochloric acid (160mL), 1 N hydrochloric acid (430mL) and absolute ethanol (1230mL). The solution was cooled in an ice bath to 5°C and 3-amino-2-chloro[(2,6-difluorobenzyl)amino]pyridine was added portionwise, followed by the addition of sodium nitrite. After stirring for 35 minutes at 5°C, aqueous methylamine was added and the solution heated at reflux for 30 minutes. The reaction was cooled in the freezer overnight, the precipitated solid collected by filtration, washed with ice water and dried overnight on a Buechner funnel to afford the title compound as the free base in quantitative yield.
   A 2-L flask fitted with a mechanical stirrer, condenser and thermometer was charged with absolute ethanol (1.0L) and heated to reflux. After removal of the heating mantle, the free base was immediately added followed by concentrated hydrochloric acid (30mL). The resulting suspension was cooled in an ice bath for 3 hours, solids collected by filtration, washed with chilled ethanol (300mL) and dried overnight in a vacuumn oven at 50°C to produce 35g (67%) of 1-(2,6-difluorobenzyl)-4-(methylamino)-1H-1,2,3-triazolo[4,5-c]pyridine hydrochloride. TLC (silica gel, EtOAc:Et₂O, 1:1) R_{f}=0.49; mp. = 297-298°C; 1H NMR (DMSO-d₆) δ = 3.07 (s, 3H,CH₃), 5.95 (s, 2H, CH₂) 7.04(d, J=6.6 Hz, 1H, C-7H), 7.19 (t,2H,C-3'H, C-5'H), 7.51 (m, 1H, C-4'H), 7.89 (d, J=6.6 Hz, 2H, C-6H), 8.88 (s,(broad), 1H, NH). Elemental analysis calculated: C, 50.09; H, 3.88; N, 22.47 Cl, 11.37; F,12.19. Found: C,50.11; H, 3.90; N,22.42; Cl,11.45; F,12.08.

### Example 6

A) Preparation of 4-amino-1-(2,6,-difluorobenzyl)-1H-1,2,3-triazolo [4,5-c]pyridine hydrochloride
Procedure: A 3-L flask equipped with a thermometer and mechanical stirrer was charged with concentrated hydrochloric acid (140mL), 1N hydrochloric acid (380mL) and absolute ethanol (1.10L) and cooled to 5°in an ice bath. 3-Amino-2-chloro-4-[(2,6-difluorobenzyl) amino]pyridine (from example 5) was added followed by the addition of sodium nitrite. The solution was stirred for 35 minutes at 5°C, ammonium hydroxide was added to pH9, and the solution extracted with chloroform (2 x 1.00 L). The combined organic extracts were dried (sodium sulphate) and concentrated to produce 4-chloro-1-(2,6-difluorobenzyl)-1H-1,2,3- triazolo[4,5-c]pyridine as a damp pink solid in quantitative yield, sufficiently pure for further reaction. 1H NMR (DMSO-d₆) δ 6.10 (s, 2H, CH₂), 7.10-7.65 (m, 3H, C-3'H, C-4'H, C-5'H), 7.95 (d, J=5.9 Hz, 1H, C-7H), 8.43 (d, J=5.9 Hz, 1H, C-6H).
The isolated solid was dissolved in ammonia saturated ethanol (2.00L), divided into two approximately equal volumes and poured into two 2-L glass-lined bombs. The bombs were heated to 120°C for 40h,¹ cooled, and the solids collected by filtration to produce 4-amino-1-(2,6-difluorobenzyl)-1H-1,2,3-triazolo[4,5-c]pyridine as the free base in 95% yield. A 2-L flask fitted with a condenser was charged with the free base and 2-methoxyethanol (1.0L), and the suspension was heated to 105°C to obtain solution. Concentrated hydrochloric acid (400mL) was added carefully through the condenser², the solution was cooled and the white solid was collected by filtration to produce 35g (80%) of 4-amino-1-(2,6-difluorobenzyl)-1H-1,2,3-triazolo[4,5-c]pyridine hydrochloride. TLC (silica gel. EtOAc:Et₂0, 1:1) R_{f}=0.10. mp. = 283-285°C; 1H NMR (DMSO-d₆) δ6.04 (s, 2H, CH₂), 7.17 (m, 2H, C-3'H, C-5'H), 7.35 (d, J=7.1 Hz, 1H, C-7H), 7.53 (m, 1H, C-4'H), 7.96 (d. J=7.1 Hz, 1H-C6H), 9.2-10.0 (broad singlet, 2H, NH₂).

| | | | | | |
|---|---|---|---|---|---|
| Elemental Analysis calculated: | C, 48.41; | H, 3.39; | N, 23.53; | Cl, 11.91; | F, 12.76. |
| Found: | C, 48.43: | H, 3.41; | N, 23.47; | Cl, 11.93; | F, 12.78. |

1. Pressure was measured - 8.4kg/cm² for the reaction.
2. The solution starts to reflux violently upon the addition of hydrochloric acid.

### Example 7

### Preparation of 1-(2,5-difluorobenzyl)-4-(methylamino)-1H-1,2,3-triazolo [4,5-c]pyridine

A Preparation of 4-[(2,5-difluorobenzyl)amino]-3-nitropyridine
   This compound was prepared in a manner analogous to example 1 A, but replacing 2-fluorobenzylamine with 2,5-difluorobenzylamine; (Aldrich).
B Preparation of 3-amino-2-chloro-4-[(2,5-difluorobenzyl)amino] pyridine
   This compound was prepared in a manner analogous to the compound of 1 B but replacing 4-[(2-fluorobenzyl)amine]-3-nitropyridine with the title compound from example 7A.
C Preparation of 1-(2,5-difluorobenzyl)-4-(methylamino)-1H-1,2,3-triazolo[4,5-c]pyridine
   This compound was prepared in an analogous manner to the compound of 1C using methylamine and 3-amino-2-chloro-4-[(2,5-difluorobenzyl) amino pyridine] as starting material. It was precipitated from a solution of the free base in EtOH with concentrated HCl. Yield 88%. mp. 273-278°.
   UV: (0.1 N HCl) λmax 275.6 (ξ15000); (pH 7 buffer) λmax 275.2 (ξ6800) λmax 304.8 (ξ9100); (0.1 N NaOH) λmax 275.2 (ξ5700) λmax 308.4 (ξ8800).

### Example 8

### Preparation of 1-benzyl-4-(methylamino)-1H-1,2,3-triazolo[4.5-c]pyridine

A Preparation of 4-(benzylamino)-3-nitropyridine.
   This compound was prepared in a manner analogous to example 1A, but replacing 2-fluorobenzylamine with benzylamine, (Aldrich).
B Preparation of 3-amino-2-chloro-4-(benzylamino)pyridine
   This compound was prepared in a manner analogous to the compound 1C, but replacing 4-(2-fluorobenzyl)amino-3-nitropyridine with the title compound from example 8A.
C Preparation of 1-benzyl-4-(methylamino)-1H-1,2,3-triazolo[4,5-c] pyridine
   This compound was prepared in an analogous manner to the compound of 1C using methylamine and 3-amino-2-chloro-4-[(benzylamino)]pyridine as starting materials. It was recrystallised from water with concentrated HCl. Yield 39%. mp. 269-273°. UV: (0.1 N Hcl) λmax 280.0 (ξ10900); (pH 7 buffer) λmax 302.8 (ξ7400); (0.1 N NaOH) λmax 306.4 (ξ8000).

### Example 9

### Preparation of 3-nitro-4-[(2,6-difluorobenzyl)amino]pyridine

- Reaction:: 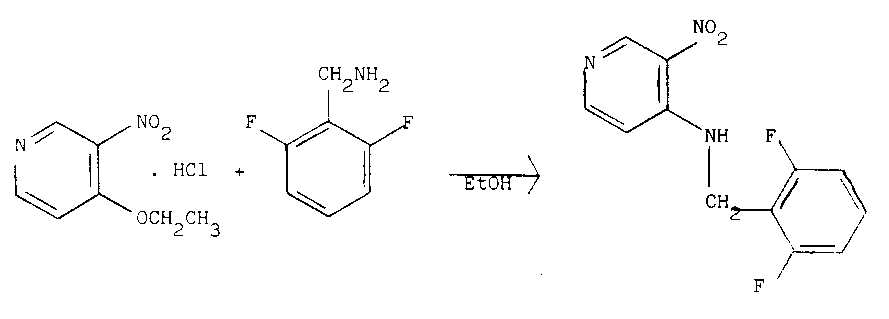
- Procedure:: A 2L Erlenmyer flask, equipped with a magnetic stirrer, was charged with sodium hydroxide and methylene chloride (800mL). With rapid stirring, 4-ethoxy-3-nitropyridine hydrochloride¹ was added portionwise over 10 minutes. The biphase mixture was stirred an additional 10 minutes, transferred to a 3L separatory funnel and the organic layer separated. The aqueous portion was extracted with methylene chloride (800mL) and the combined organic extracts were concentrated to dryness in vacuo to yield the free base as a tan solid (175g, 104%) which was used without further purification.
A one-neck 3L flask, equipped with a reflux condenser, magnetic stirrer, and nitrogen inlet was charged with freshly prepared 4-ethoxy-3-nitropyridine, 2,6-difluorobenzylamine² and absolute ethanol (1.9L). The solution was heated at reflux for 20h, cooled slightly, poured into an Erlenmyer flask and refrigerated overnight. The resulting yellow precipitate was collected by filtration, washed with chilled ethanol (150mL) and dried on a Buechner funnel to yield 234g (88.4%) of 3-nitro-4-[(2,6-difluorobenzyl)amino]pyridine: TLC (silica gel, EtOAc/Hexane, 1:1) R_{f}=0.31; MP= 148-149°C; 1H NMR (DMSO-d₆) δ 5.74 (d,J=5.7 Hz, 2H, CH₂), 7.01 (d, J=6.3 Hz, 1H, C-5H), 7.14 (m, 2H, C-3'H and C-5'H), 7.44 (m, 1H, C-4'H), 8.32 (d, J=6.2 Hz, 1H, C-6H), 8.65 (t, J=6.0 Hz, 1H, NH) and 9.03 (s, 1H, C-2H). Elemental Analysis calculated C, 54.35; H, 3.42; N, 15.84; F, 14.33; Found, C, 54.40; H, 3.44; N, 15.85; F, 14.21.

1. 4-Ethoxy-3-nitropyridine hydrochloride was prepared according to the literature produced of E.R Lavagnio, et al (J.Heterocyclic.Chem, 1986 23, 669). The compound is more stable as the hydrochloride salt and is neutralised immediately before use.
2. 2,6-Difluorobenzylamine is commercially available but was prepared from a BN₃. THF reduction of 2,6-difluorobenzonitrile. The product contained 23% (wt) of 1-butanol by ¹H NMR and this is factored into the quantity of 2,6-difluorobenzylamine employed.

### Example 10

### Preparation of 4-(cyclopropylamino)-1-(2,6-difluorobenzyl)-1H-1,2,3-triazolo[4,5-c]pyridine.

This compound was prepared in an analogous manner to that of example 5 with the replacement of methylamine in example 5C with cyclopropylamine. It was precipitated from the free base in EtOH with concentrated HCl. Yield 70%, mp > 260°C (dec). UV:(0.1N HCl) λmax 286 (ξ14200); (pH 7 buffer) λmax 306 (ξ9900); (0.1 N NaOH) λmax 309 (ξ10000).

### Pharmacological Activity

The anticonvulsant activity of certain compounds of the present invention were determined by a standard maximal electroshock test (MES); that described by L.A.Woodbury and V.D.Davenport, Arch. Int.Pharmacodyn, 1952 92 97.

| COMPOUND OF EXAMPLE NO | SALT | ED50.I.P (mg/kg) | ED50.P.O (mg/kg) |
|---|---|---|---|
| 1 | HCl | 4.5 | 4.4 |
| 2 | HCl | 4.8 | 6.3 |
| 3 | HCl | 8.3 | 10.3 |
| 4 | HCl | 6.3 | 6.6 |
| 5 | HCl | 7.8 | 5.3 |
| 6 | HCl | 6.3 | 3.7 |
| 7 | HCl | 5.0 | 4.3 |
| 8 | HCl | 7.8 | 8.9 |
| 10 | HCl | 4.1 | 8.1 |

### FORMULATION EXAMPLES

### I - Formulation

| | |
|---|---|
| Compound | 25mg |
| Corn starch | 45mg |
| Polyvinylpyrrolidone | 6mg |
| Stearic acid | 12mg |
| Magnesium stearate | 2mg |
| Lactose qs to | 300mg |

The compound is finely ground and intimately mixed with the powdered excipients lactose and corn starch. The powders are wetted with a solution of polyvinylpyrrolidone dissolved in purified water and denatured alcohol to form granules. The granules are dried and mixed with the powdered stearic acid and magnesium stearate. The formulation is then compressed into tablets weighing approximately 300mg each.

### II - Capsule

| | |
|---|---|
| Active ingredient | 25mg |
| Corn starch | 45mg |
| Stearic acid | 12mg |
| Lactose qs to | 300mg |

The finely ground active ingredient is mixed with the powdered exipients lactose and corn starch, and stearic acid and filled into hard-shell gelatin capsules.

### III - Suppository

| | |
|---|---|
| Active ingredient | 25mg |
| Cocoa butter | 1975mg |

The cocoa butter is heated to melting and the active ingredient is dispersed by thorough mixing. The mass is then formed into suppositories weighing approximately 2,000mg each.

### IV - Injection

| | |
|---|---|
| Active ingredient | 25mg |
| Sodium chloride | 0.9% |
| Preservative | 0.1% |
| Hydrochloric acid or sodium hydroxide | as need for pH adjustment |
| Water for injection | qs to 2-3ml. |

The active ingredient, sodium chloride, and preservative are dissolved in a portion of the water for injection. The pH of the solution is adjusted with hydrochloric acid or sodium hydroxide. Water for injection is added to final volume and the solution is thoroughly mixed. The solution is sterilized by filtration through a 0.22 micrometer membrane filter and aseptically filled into sterile containers.

### V - Syrup

| | |
|---|---|
| Active ingredient | 15mg |
| Glycerin | 500mg |
| Sucrose | 3500mg |
| Flavouring agent | qs |
| Colouring agent | qs |
| Preserving agent | 0.1% |
| Purified water | qs to 5ml |

The active ingredient and sucrose are dissolved in the glycerin and a portion of the purified water. The preserving agent is dissolved in another portion of hot purified water, and then the colouring agent is added and dissolved. The two solutions are mixed and cooled before the flavouring agent is added. Purified water is added to final volume. The resulting syrup is thoroughly mixed.

## Claims (Claims for the following Contracting State(s): AT, BE, CH, DE, DK, FR, GB, IT, LI, LU, NL, SE)

1. A compound of formula (I) 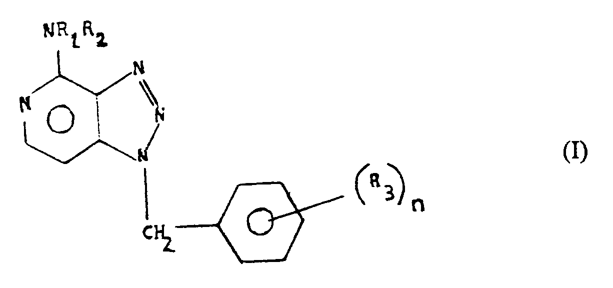 and salts thereof
wherein R₁ and R₂ are the same or different and are each selected from hydrogen, C₁₋₄ straight alkyl, C₃₋₄ branched alkyl, cyclo C₃₋₄ alkyl, and cyclopropylmethyl; R₃ is hydrogen or halo and n is either 1 or 2.

2. A compound as claimed in claim 1 or a salt thereof wherein R₃ is fluoro.

3. A compound according to claims 1 and 2 and salts thereof wherein -(R₃)ₙ represents any of the following: 2-fluoro, 3-fluoro, 4-fluoro, 2,6-difluoro; 2,5-difluoro; 2,4-difluoro; 3,4-difluoro and 3,5-difluoro.

4. A compound according to claims 1 to 3 and salts thereof wherein -(R₃)ₙ represents 2,6-difluoro.

5. A compound according to claim 1 selected from the following:
1-(2-fluorobenzyl)-4-(methylamino)-1H-1,2,3-triazolo[4,5-c]pyridine;
4-(cyclopropylamino)-1-(2-fluorobenzyl)-1H-1,2,3-triazolo[4,5-c]pyridine;
4-(ethylamino)-1-(2-fluorobenzyl)-1H-1,2,3-triazolo[4,5-c]pyridine;
4-(dimethylamino)-1-(2-fluorobenzyl)-1H-1,2,3-triazolo[4,5-c]pyridine;
1-(2,6-difluorobenzyl)-4-(methylamino)-1H-1,2,3-triazolo[4,5-c]pyridine;
4-amino-1-(2,6-difluorobenzyl)-1H-1,2,3-triazolo[4,5-c]pyridine;
1-(2,5-difluorobenyzl)-4-(methylamino)-1H-1,2,3-triazolo[4,5-c]pyridine and
1-benzyl-4-(methylamino)-1H-1,2,3-triazolo[4,5-c]pyridine,
and salts thereof.

6. 4-Amino-1-(2,6-difluorobenzyl)-1H-1,2,3-triazolo[4,5-c]pyridine and salts thereof.

7. A salt of a compound according to claims 1 to 6.

8. A pharmaceutically acceptable salt according to claims 1 to 7.

9. A salt of 4-amino-1-(2,6-difluorobenzyl)-1H-1,2,3-triazolo[4,5-c]pyridine.

10. A pharmaceutically acceptable salt of 4-amino-1-(2,6-difluorobenzyl)-1H-1,2,3-triazolo[4,5-c]pyridine.

11. The hydrochloride salt of 4-amino-1-(2,6-difluorobenzyl)-1H-1,2,3-triazolo[4,5-c]pyridine.

12. A compound or salt according to claims 1 to 11 for use in therapy.

13. A process for the preparation of a compound as defined in claims 1 to 11 which comprises the reaction of an amine HNR₁R₂ with a compound of formula (II) where R₁, R₂, R₃ and n are as defined in claim 1, and X is a leaving group.

14. A process according to claim 12 for the preparation of 1-(2,6-difluorobenzyl)-4-(methylamino)-1H-1,2,3-triazolo[4,5-c]pyridine and salts thereof.

15. A process according to claim 12 for the preparation of 4-amino-1-(2,6-difluoro benzyl)-1H-1,2,3-triazolo[4,5-c]pyridine.

16. A process for the preparation or 4-amino-1-(2,6-difluorobenzyl)-1H-1,2,3-triazolo[4,5-c]pyridine comprising the reaction of 4-chloro-1-(2,6-difluorobenzyl)-1H-1,2,3-trizolo[4,5-c]pyridine with ammonia.

17. A compound of formula (II) and salts thereof 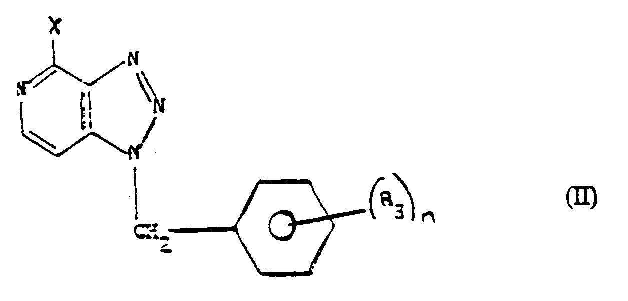 wherein X is any of halo, C₁₋₆alkylthio, C₆₋₁₀arylthio, C₇₋₁₂ aralkylthio, C₁₋₄ alkyl, phenyl, benzyl, phenylethyl, napthylmethyl and substituted sulphonyl or sulphinyl;
R₃ is hydrogen or halo and n is either 1 or 2

18. A compound or salt as claimed in claim 17 wherein X is chloro.

19. The use of a compound or salt as described in claim 1 for the preparation of a medicament for use in the treatment of a CNS disorder.

20. Use according to claim 19 wherein the compound is 4-amino-1-(2,6-difluorobenzyl)-1H-1,2,3-triazolo[4,5-c]pyridine.

21. Use according to claim 19 wherein the salt is the hydrochloride salt of 4-amino-1-(2,6-difluorobenzyl)-1H-1,2,3-triazolo[4,5-c]pyridine and the CNS disorder is epilepsy.

22. A pharmaceutical formulation comprising a compound or salt as defined in claims 1 to 11 together with a pharmaceutically acceptable carrier.

23. A pharmaceutical formulation comprising 4-amino-1-(2,6-difluorobenzyl)-1H-1,2,3-triazolo (4,5-c)pyridine or a salt thereof together with a pharmaceutically acceptable carrier.

## Claims (Claims for the following Contracting State(s): ES, GR)

1. A process for the preparation of a compound of formula (I) 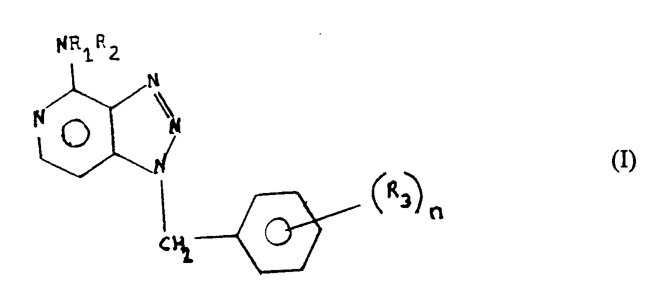 and salts thereof
where R₁ and R₂ may be the same or different and are each selected from hydrogen, C₁₋₄ straight alkyl, C₃₋₄ branched alkyl, cyclo C₃₋₄ alkyl and cyclopropylmethyl;
R₃ is hydrogen or halo and n is either 1 or 2
which comprises the reaction of an amine HNR₁R₂ with a compound of formula (II) 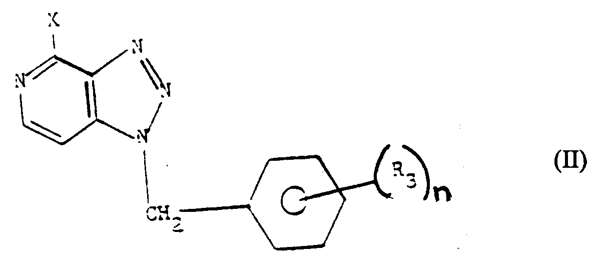 wherein R₁, R₂, R₃ and n are as defined and X is a leaving group, and optionally converting into a salt thereof.

2. A process according to claim 1 wherein X is any of: halogen, C₁₋₆alkylthio, C₆₋₁₀arylthio, C₇₋₁₂aralkylthio, C₁₋₄alkyl, phenyl, benzyl, phenylethyl, naphthylmethyl and substituted sulphonyl or sulphinyl.

3. A process according to claims 1 and 2 wherein X is chloro.

4. A process according to claims 1 to 3 wherein -(R₃)ₙ represents any of: 2-fluoro, 3-fluoro, 4-fluoro; 2,6-difluoro: 2,5-difluoro; 2,4-difluoro; 2,3-difluoro: 3,4-difluoro and 3,5-difluoro.

5. A process according to claims 1 to 3 wherein -(R₃)ₙ is 2,6-difluoro.

6. A process according to claims 1 to 4 wherein the reaction takes place in a polar solvent.

7. A process according to claims 1 to 5 wherein the reaction takes place between 0° C to 180°C.

8. A process according to claims 1 to 7 for preparing a salt of a compound as described in claim 1.

9. A process according to claims 1 to 7 for preparing a pharmaceutically acceptable salt of a compound as described in claim 1.

10. A process according to claims 1 to 6 for preparing 1-(2,6- difluoro-benzyl)-4-(methylamino)-1H-1,2,3-triazolo[4,5-c]pyridine.

11. A process according to claims 1 to 7 for preparing 4-amino-1-(2,6-difluorobenzyl)-1H-1,2,3-triazolo[4,5-c]pyridine.

12. A process according to claims 1 to 7 for preparing a salt of 4-amino-1-(2,6-difluorobenzyl)-1H-1,2,3-triazolo[4,5-c]pyridine.

13. A process according to claims 1 to 7 for preparing a pharmaceutically acceptable salt of 4-amino-1-(2,6-difluorobenzyl)-1H-1,2,3-triazolo[4,5-c]pyridine.

14. A process according to claims 1 to 7 for preparing the hydrochloride salt of 4-amino-1-(2,6-difluorobenzyl)-1H-1,2,3-triazolo[4,5-c]pyridine.

15. A process for the preparation of 4-amino-1-(2,6-difluorobenzyl)-1H-1,2,3-triazolo[4,5-c]pyridine comprising the reaction of 4-chloro-1-(2,6-difluorobenzyl)-1H-1,2,3-triazolo[4,5-c]pyridine with ammonia.

16. A process for preparing the hydrochloride salt of 4-amino-1-(2,6-difluorobenzyl)-1H-1,2,3-triazolo[4,5-c]pyridine comprising the reaction of 4-amino-1-(2,6-difluorobenzyl)-1H-1,2,3-triazolo[4,5-c]pyridine with hydrochloric acid.

17. A process for the production of a compound of formula (II) 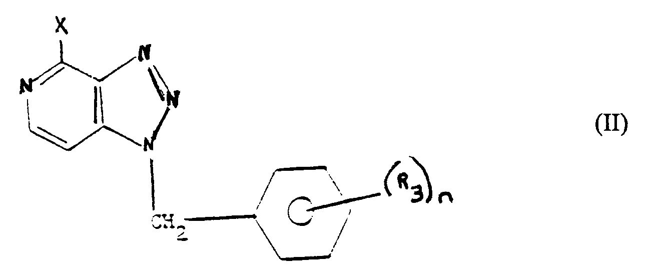 and salts thereof
wherein X is any of halo, C₁₋₆alkylathio, C₆₋₁₀arylthio, C₇₋₁₂aralkylthio, C₁-₄alkyl, phenyl, benzyl, phenylethyl, napthylmethyl and substituted sulphonyl or sulphinyl;
R₃ is hydrogen or halo and n is either 1 or 2
which process comprises the reaction of a compound of formula (III) 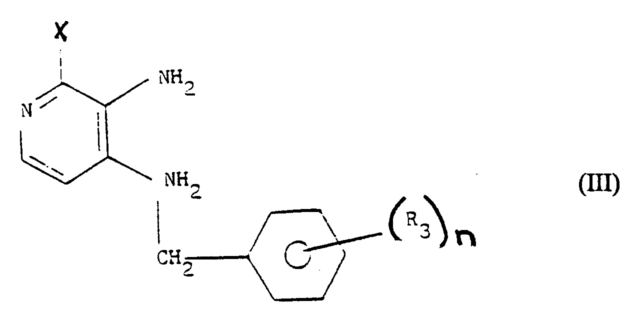 wherein X, R₃ and n are as hereindefined, with sodium nitrite in hydrohalic acid.

## Patentansprüche (Patentansprüche für folgende(n) Vertragsstaat(en): AT, BE, CH, DE, DK, FR, GB, IT, LI, LU, NL, SE)

1. Verbindung der Formel (I) 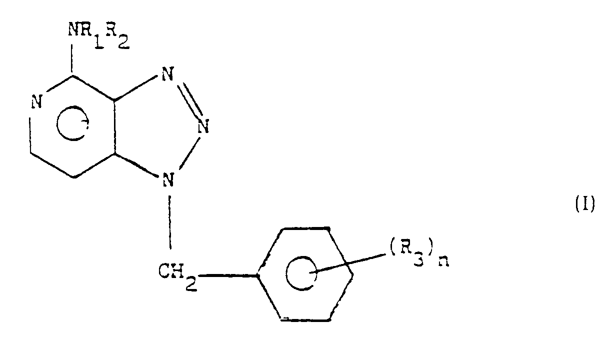 und Salze davon,
worin R₁ und R₂ gleich oder verschieden sind und jeweils aus Wasserstoff, C₁₋₄-geradkettiges Alkyl, C₃₋₄-verzweigtes Alkyl, Cyclo-C₃₋₄-alkyl und Cyclopropylmethyl gewählt sind: R₃ Wasserstoff oder Halogen und n entweder 1 oder 2 bedeuten.

2. Verbindung nach Anspruch 1 oder ein Salz davon, wobei R₃ Fluor ist.

3. Verbindung nach den Ansprüchen 1 und 2 und Salze davon, wobei -(R₃)ₙ eine der folgenden Bedeutungen hat: 2-Fluor, 3-Fluor, 4-Fluor, 2,6-Difluor, 2,5-Difluor, 2,4-Difluor, 3,4-Difluor und 3,5-Difluor.

4. Verbindung nach den Ansprüchen 1 bis 3 und Salze davon, wobei -(R₃)ₙ die Bedeutung 2,6-Difluor hat.

5. Verbindung nach Anspruch 1, gewählt aus den folgenden:
1-(2-Fluorbenzyl)-4-(methylamin)-1H-1,2,3-triazolo[4,5-c]pyridin;
4-(Cyclopropylamin)-1-(2-fluorbenzyl)-1H-1,2,3-triazolo[4,5-c]pyridin;
4-(Ethylamin)-1-(2-fluorbenzyl)-1H-1,2,3-triazolo[4,5-c]pyridin;
4-(Dimethylamin)-1-(2-fluorbenzyl)-1H-1,2,3-triazolo[4,5-c]pyridin;
1-(2,6-Difluorbenzyl)-4-(methylamin)-1H-1,2,3-triazolo[4,5-c]pyridin;
4-Amino-1-(2,6-difluorbenzyl)-1H-1,2,3-triazolo[4,5-c]pyridin;
1-(2,5-Difluorbenzyl)-4-(methylamino)-1H-1,2,3-triazolo[4,5-c]pyridin und
1-Benzyl-4-(methylamin)-1H-1,2,3-triazolo[4,5-c]pyridin,
und Salze davon.

6. 4-Amino-1-(2,6-difluorbenzyl)-1H-1,2,3-triazolo[4,5-c]pyridin und Salze davon.

7. Salz einer Verbindung nach den Ansprüchen 1 bis 6.

8. Pharmazeutisch annehmbares Salz nach den Ansprüchen 1 bis 7.

9. Salz von 4-Amino-1-(2,6-difluorbenzyl)-1H-1,2,3-triazolo[4,5-c]pyridin.

10. Pharmazeutisch annehmbares Salz von 4-Amino-1-(2,6-difluorbenzyl)-1H-1,2,3-triazolo[4,5-c]pyridin.

11. Hydrochloridsalz von 4-Amino-1-(2,6-difluorbenzyl)-1H-1,2,3-triazolo[4,5-c]pyridin.

12. Verbindung oder Salz nach den Ansprüchen 1 bis 11 zur Verwendung in der Therapie.

13. Verfahren zur Herstellung einer wie in den Ansprüchen 1 bis 11 definierten Verbindung, umfassend die Umsetzung eines Amins HNR₁R₂ mit einer Verbindung der Formel (II) 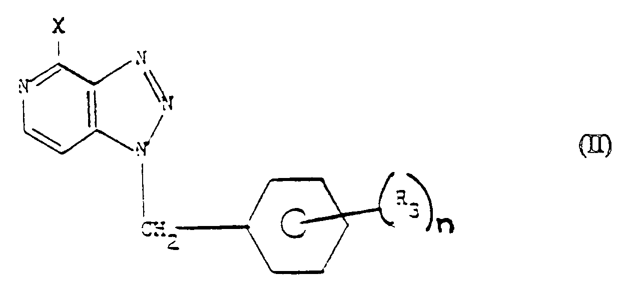 worin R₁, R₂, R₃ und n wie in Anspruch 1 definiert sind und X eine Abgangsgruppe ist.

14. Verfahren nach Anspruch 12 zur Herstellung von 1-(2,6-Difluorbenzyl)-4-(methylamin)-1H-1,2,3-triazolo[4,5-c]pyridin und Salzen davon.

15. Verfahren nach Anspruch 12 zur Herstellung von 4-Amino-1-(2,6-difluorbenzyl)-1H-1,2,3-triazolo[4,5-c]pyridin.

16. Verfahren zur Herstellung von 4-Amino-1-(2,6-difluorbenzyl)-1H-1,2,3-triazolo[4,5-c]pyridin, umfassend die Umsetzung von 4-Chlor-1-(2,6-difluorbenzyl)-1H-1,2,3-triazolo[4,5-c]pyridin mit Ammoniak.

17. Verbindung der Formel (II) und Salze davon 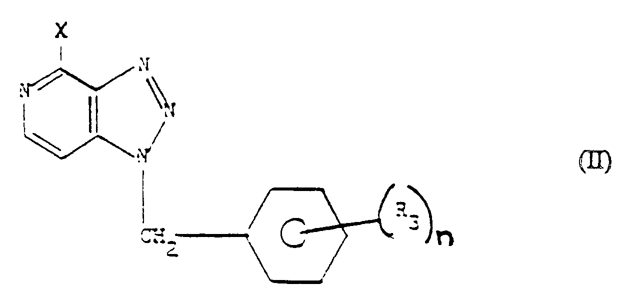 worin X eines aus Halogen, C₁₋₆-Alkylthio, C₆₋₁₀-Arylthio, C₇₋₁₂-Aralkylthio, C₁₋₄-Alkyl, Phenyl, Benzyl, Phenylethyl, Naphthylmethyl und substituiertes Sulfonyl oder Sulfinyl ist; R₃ Wasserstoff oder Halogen ist und n entweder 1 oder 2 ist.

18. Verbindung oder Salz nach Anspruch 17, wobei X Chlor ist.

19. Verwendung einer Verbindung oder eines Salzes wie in Anspruch 1 beschrieben zur Herstellung eines Arzneimittels zur Verwendung bei der Behandlung einer CNS-Störung.

20. Verwendung nach Anspruch 19, wobei die Verbindung 4-Amino-1-(2,6-difluorbenzyl)-1H-1,2,3-triazolo[4,5-c]pyridin ist.

21. Verwendung nach Anspruch 19, wobei das Salz das Hydrochloridsalz von 4-Amino-1-(2,6-difluorbenzyl)-1H-1,2,3-triazolo[4,5-c]pyridin ist und die CNS-Störung Epilepsie ist.

22. Pharmazeutische Zubereitung, umfassend eine Verbindung oder ein Salz wie in den Ansprüchen 1 bis 11 definiert, zusammen mit einem pharmazeutisch annehmbaren Träger.

23. Pharmazeutische Zubereitung, umfassend 4-Amino-1-(2,6-difluorbenzyl)-1H-1,2,3-triazolo[4,5-c]pyridin oder ein Salz davon, zusammen mit einem pharmazeutisch annehmbaren Träger.

## Patentansprüche (Patentansprüche für folgende(n) Vertragsstaat(en): ES, GR)

1. Verfahren zur Herstellung einer Verbindung der Formel (I) 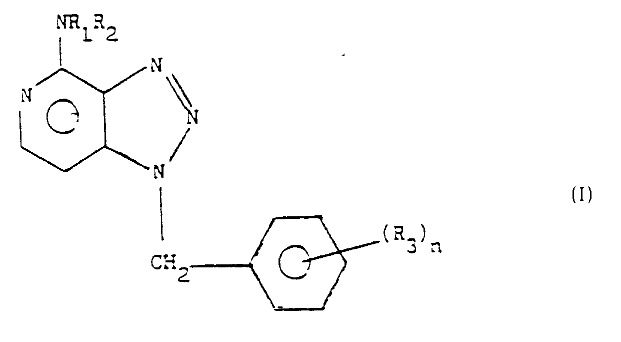 und von Salzen davon
worin R₁ und R₂ gleich oder verschieden sein können und jeweils aus Wasserstoff, C₁₋₄-geradkettiges Alkyl, C₃₋₄-verzweigtkettiges Alkyl, Cyclo-C₃₋₄-alkyl und Cyclopropylmethyl gewählt sind; R₃ Wasserstoff oder Halogen und n entweder 1 oder 2 bedeuten.
umfassend die Umsetzung eines Amins HNR₁R₂ mit einer Verbindung der Formel (II) 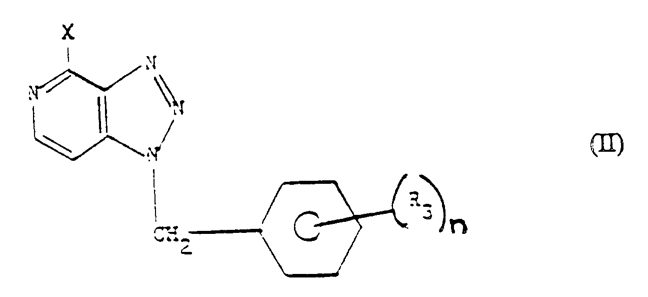 worin R₁, R₂, R₃ und n wie definiert sind und X eine Abgangsgruppe ist, und wahlweise Umwandeln in ein Salz davon.

2. Verfahren nach Anspruch 1, wobei X eines aus Halogen, C₁₋₆-Alkylthio, C₆₋₁₀-Arylthio, C₇₋₁₂-Aralkylthio, C₁₋₄-Alkyl, Phenyl, Benzyl, Phenylethyl, Naphthylmethyl und substituiertes Sulfonyl oder Sulfinyl ist.

3. Verfahren nach den Ansprüchen 1 und 2, wobei X Chlor ist.

4. Verfahren nach den Ansprüchen 1 bis 3, wobei -(R₃)ₙ eine der folgenden Bedeutungen hat: 2-Fluor, 3-Fluor, 4-Fluor, 2,6-Difluor, 2,5-Difluor, 2,4-Difluor, 3,4-Difluor und 3,5-Difluor.

5. Verfahren nach den Ansprüchen 1 bis 3, wobei -(R₃)ₙ die Bedeutung 2,6-Difluor hat.

6. Verfahren nach den Ansprüchen 1 bis 4, wobei die Umsetzung in einem polaren Lösungsmittel stattfindet.

7. Verfahren nach den Ansprüchen 1 bis 5, wobei die Umsetzung zwischen 0°C bis 180°C stattfindet.

8. Verfahren nach den Ansprüchen 1 bis 7 zur Herstellung eines Salzes einer wie in Anspruch 1 beschriebenen Verbindung.

9. Verfahren nach den Ansprüchen 1 bis 7 zur Herstellung eines pharmazeutisch annehmbaren Salzes einer wie in Anspruch 1 beschriebenen Verbindung.

10. Verfahren nach den Ansprüchen 1 bis 6 zur Herstellung von 1-(2,6-Difluorbenzyl)-4-(methylamino)-1H-1,2,3-triazolo[4,5-c]pyridin.

11. Verfahren nach den Ansprüchen 1 bis 7 zur Herstellung von 4-Amino-1-(2,6-difluorbenzyl)-1H-1,2,3-triazolo[4,5-c]pyridin.

12. Verfahren nach den Ansprüchen 1 bis 7 zur Herstellung eines Salzes von 4-Amino-1-(2,6-difluorbenzyl)-1H-1,2,3-triazolo[4,5-c]pyridin.

13. Verfahren nach den Ansprüchen 1 bis 7 zur Herstellung eines pharmazeutisch annehmbaren Salzes von 4-Amino-1-(2,6-difluorbenzyl)-1H-1,2,3-triazolo[4,5-c]pyridin.

14. Verfahren nach den Ansprüchen 1 bis 7 zur Herstellung des Hydrochloridsalzes von 4-Amino-1-(2,6-difluorbenzyl)-1H-1,2,3-triazolo[4,5-c]pyridin.

15. Verfahren zur Herstellung von 4-Amino-1-(2,6-difluorbenzyl)-1H-1,2,3-triazolo[4,5-c]pyridin, umfassend die Umsetzung von 4-Chlor-1-(2,6-difluorbenzyl)-1H-1,2,3-triazolo[4,5-c]pyridin mit Ammoniak.

16. Verfahren zur Herstellung des Hydrochloridsalzes von 4-Amino-1-(2,6-difluorbenzyl)-1H-1,2,3-triazolo[4,5-c]pyridin, umfassend die Umsetzung von 4-Amino-1-(2,6-difluorbenzyl)-1H-1,2,3-triazolo[4,5-c]pyridin mit Chlorwasserstoffsäure.

17. Verfahren zur Herstellung einer Verbindung der Formel (II) 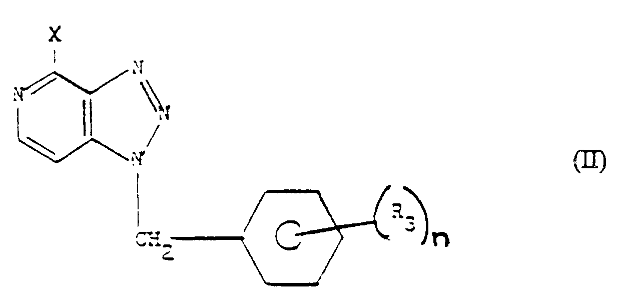 und von Salzen davon,
worin X eines aus Halogen, C₁₋₆-Alkylthio, C₆₋₁₀-Arylthio, C₇₋₁₂-Aralkylthio, C₁₋₄-Alkyl, Phenyl, Benzyl, Phenylethyl, Naphthylmethyl und substituiertes Sulfonyl oder Sulfinyl ist;
R₃ Wasserstoff oder Halogen ist und n entweder 1 oder 2 ist,
welches Verfahren die Umsetzung einer Verbindung der Formel (III) 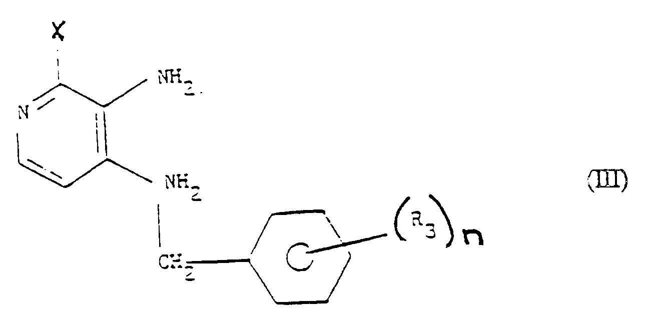 worin X, R₃ und n wie vorangehend definiert sind, mit Natriumnitrit in einer Halogenwasserstoffsäure umfaßt.

## Revendications (Revendications pour l'(les) Etat(s) contractant(s) suivant(s): AT, BE, CH, DE, DK, FR, GB, IT, LI, LU, NL, SE)

1. Un Composé de formule (I) : 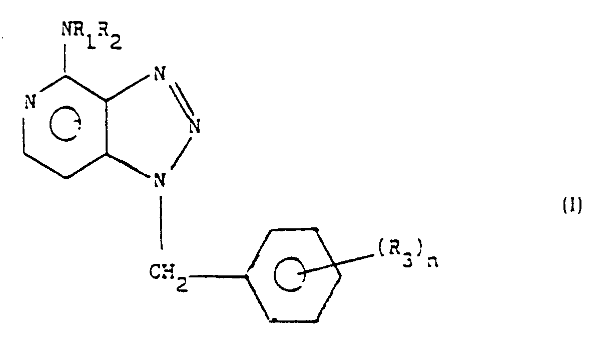 et les sels de celui-ci,
dans laquelle R¹ et R² sont identiques ou différents et sont choisis chacun dans le groupe constitué par hydrogène, un groupement alkyle en C₁₋₄ à chaîne droite, un groupement alkyle en C₃₋₄ à chaîne ramifiée, un groupement cyclo-C₃₋₄-alkyle et un groupement cyclopropylméthyle ; R3 est un hydrogène ou un halogène et n est 1 ou 2.

2. Composé selon la revendication 1 ou sel de celui-ci dans lequel R3 est un groupement fluoro.

3. Composé selon les revendications 1 et 2 et sels de celui-ci, dans lequel -(R₃)ₙ a l'une des significations suivantes : 2-fluoro, 3-fluoro, 4-fluoro, 2,6-difluoro ; 2,5-difluoro ; 2,4-difluoro 3,4-difluoro et 3,5-difluoro.

4. Composé selon les revendications 1 à 3 et sels de celui-ci, dans lequel -(R₃)ₙ représente 2,6-difluoro.

5. Composé selon la revendication 1 choisi parmi les suivants :
1-(2-fluorobenzyl)-4-(méthylamino)-1H-1,2,3-triazolo[4,5-c]pyridine ;
4-(cyclopropylamino)-1-(2-fluorobenzyl)-1H-1,2,3-triazolo[4,5-c]pyridine ;
4-(éthylamino)-1-(2-fluorobenzyl)-1H-1,2,3-triazolo[4,5-c]pyridine ;
4-(diméthylamino)-1-(2-fluorobenzyl)-1H-1,2,3-triazolo[4,5-c]pyridine ;
1-(2,6-difluorobenzyl)-4-(méthylamino)-1H-1,2,3-triazolo[4,5-c]pyridine ;
4-amino-1-(2,6-difluorobenzyl)-1H-1,2,3-triazolo[4,5-c]pyridine ;
1-(2,5-difluorobenzyl)-4-(méhtylamino)-1H-1,2,3-triazolo[4,5-c]pyridine et
1-benzyl-4-(méthylamino)-1H-1,2,3- triazolo[4,5-c]pyridine,
et sels de ceux-ci.

6. 4-amino-1-(2,6-difluorobenzyl)-1H-1,2,3-triazolo[4,5-c]pyridine et sels de celle-ci.

7. Sel d'un composé selon les revendications 1 à 6.

8. Sel pharmaceutiquement acceptable selon les revendications 1 à 7.

9. Sel de la 4-amino-1-(2,6-difluorobenzyl)-1H-1,2,3-triazolo [4,5-c]pyridine.

10. Sel pharmaceutiquement acceptable de la 4-amino-1-(2,6-difluorobenzyl)-1H-1,2,3-triazolo-[4,5-c]pyridine.

11. Chlorhydrate de la 4-amino-1-(2,6-difluorobenzyl)-1H-1,2,3-triazolo [4,5-c]pyridine.

12. Composé ou sel selon les revendications 1 à 11 destiné à un usage thérapeutique.

13. Procédé de préparation d'un composé tel que défini dans les revendications 1 à 11, qui comprend la réaction d'une amine HNR₁R₂ avec un composé de formule (II) 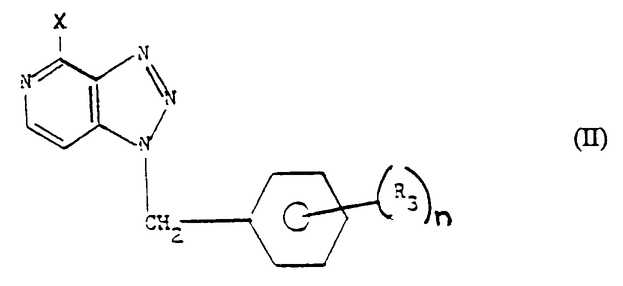 dans laquelle R₁, R₂ R₃ et n sont tels que définis dans la revendication 1, et X est un groupement partant.

14. Procédé selon la revendication 12 pour la préparation de la 1-(2,6-difluorobenzyl)-4-(méthylamino)-1H-1,2,3-triazolo[4,5-c]pyridine et des sels de celle-ci.

15. Procédé selon la revendication 12 pour la préparation de la 4-amino-1-(2,6-difluorobenzyl))-1H-1,2,3-triazolo[4,5-c]pyridine.

16. Procédé de préparation de la 4-amino-1-(2,6-difluorobenzyl))-1H-1,2,3-triazolo[4,5-c] pyridine, comprenant la réaction de la 4-chloro-1-(2,6-difluorobenzyl))-1H-1,2,3-triazolo[4,5-c]pyridine avec l'ammoniac;

17. Composé de formule (II) et sels de celui-ci 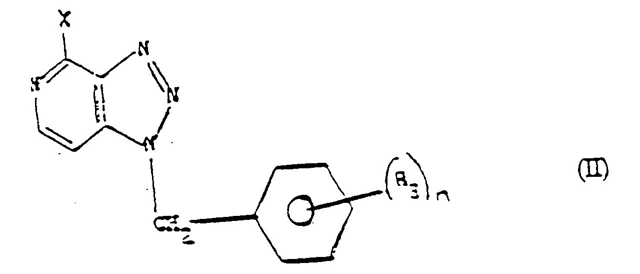 dans laquelle X est un membre quelconque du groupe comprenant un halogène, alkylthio en C₁₋₆, arylthio en C₆₋₁₀, aralkylthio en C₇₋₁₂, alkyle en C₁₋₄, phényle, benzyle, phényléthyle, naphtylméthyle et sulfonyle ou sulfinyle substitué ;
R₃ est un hydrogène ou un halogène et n est 1 ou 2.

18. Composé ou sel tel que revendiqué dans la revendication 17 dans laquelle X est un chlore.

19. Utilisation d'un composé ou d'un sel décrit dans la revendication 1 dans la préparation d'un médicament destiné au traitement d'un trouble du système nerveux central.

20. Utilisation selon la revendication 19 dans laquelle le composé est la 4-amino-1-(2,6-difluorobenzyl))-1H-1,2,3-triazolo[4,5-c]pyridine.

21. Utilisation selon la revendication 19 dans laquelle le sel est un chlorhydrate de la 4-amino-1-(2,6-difluorobenzyl))-1H-1,2,3-triazolo[4,5-c]pyridine et le trouble du système nerveux central est l'épilepsie.

22. Composition pharmaceutique comprenant un composé ou un sel tel que défini dans les revendications 1 à 11 avec un véhicule pharmaceutiquement acceptable.

23. Composition pharmaceutique comprenant la 4-amino-1-(2,6-difluorobenzyl))-1H-1,2,3-triazolo- [4,5-c]pyridine ou un sel de celle-ci avec un véhicule pharmaceutiquement acceptable.

## Revendications (Revendications pour l'(les) Etat(s) contractant(s) suivant(s): ES, GR)

1. Procédé de préparation d'un composé de formule (I) : 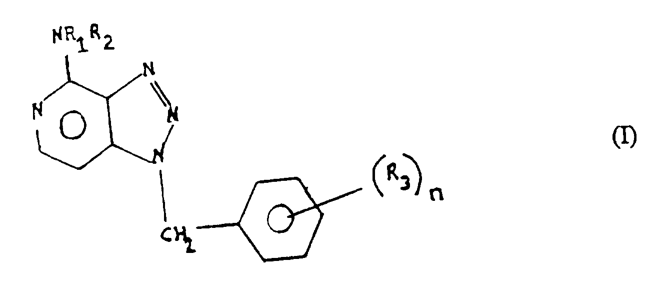 et des sels de celui-ci
dans laquelle R¹ et R² sont identiques ou différents et sont choisis chacun dans le groupe constitué par un hydrogène, un groupement alkyle en C₁₋₄ à chaîne droite, un groupement alkyle en C₃₋₄ à chaîne ramifiée, un groupement cyclo-C₃₋₄alkyle et un groupement cyclopropylméthyle ;
R3 est un hydrogène ou un halogène et n est 1 ou 2
qui comprend la réaction d'une amine HNR₁R₂ avec un composé de formule (II) 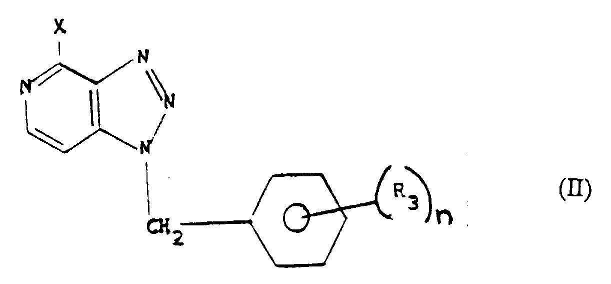 dans laquelle R₁, R₂, R₃ et n sont tels que définis et X est un groupement partant, et éventuellement la conversion en un sel de celui-ci.

2. Procédé selon la revendication 1, dans lequel X est un membre quelconque du groupe comprenant un halogène, un groupement alkylthio en C₁₋₆, arylthio en C₆₋₁₀, arakylthio en C₇₋₁₂, alkyle en C₁₋₄, phényle, benzyle, phényléthyle, naphtylméthyle et sulfonyle ou sulfinyle substitué .

3. Procédé selon les revendications 1 et 2 dans lequel X est un chlore.

4. Procédé selon les revendications 1 à 3 dans lequel -(R₃)ₙ a l'une des significations suivantes : 2-fluoro, 3-fluoro, 4-fluoro, 2,6-difluoro ; 2,5-difluoro ; 2,4-difluoro ; 2,3-difluoro ; 3,4-difluoro et 3,5-difluoro.

5. Procédé selon les revendications 1 à 3 dans lequel -(R₃)ₙ a la signification 2,6-difluoro.

6. Procédé selon les revendications 1 à 4 dans lequel la réaction a lieu dans un solvant polaire.

7. Procédé selon les revendications 1 à 5 dans lequel la réaction a lieu entre 0°C et 180°C.

8. Procédé selon les revendications 1 à 7 pour la préparation d'un sel d'un composé tel que décrit dans la revendication 1.

9. Procédé selon les revendications 1 à 7 pour la préparation d'un sel pharmaceutiquement acceptable d'un composé tel que décrit dans la revendication 1.

10. Procédé selon les revendications 1 à 6 pour la préparation de la 1-(2,6-difluorobenzyl)-4-(méthylamino)-1H-1,2,3-triazolo[4,5-c]pyridine.

11. Procédé selon les revendications 1 à 7 pour la préparation de la 4-amino-1-(2,6-difluorobenzyl)-1H-1,2,3-triazolo[4,5-c]pyridine.

12. Procédé selon les revendications 1 à 7 pour la préparation d'un sel de la 4-amino-1-(2,6-difluorobenzyl)-1H-1,2,3-triazolo[4,5-c]pyridine.

13. Procédé selon les revendications 1 à 7 pour la préparation d'un sel pharmaceutiquement acceptable de 4-amino-1-(2,6-difluorobenzyl)-1H-1,2,3-triazolo-[4,5-c]pyridine.

14. Procédé selon les revendications 1 à 7 pour la préparation du chlorhydrate de 4-amino-1-(2,6-difluorobenzyl)-1H-1,2,3-triazolo[4,5-c]pyridine.

15. Procédé pour la préparation de la 4-amino-1-(2,6-difluorobenzyl)-1H-1,2,3-triazolo[4,5-c]pyridine, comprenant la réaction de la 4-chloro-1-(2,6-difluorobenzyl)-1H-1,2,3-triazolo[4,5-c]pyridine avec l'ammoniac.

16. Procédé pour la préparation du chlorhydrate de la 4-amino-1-(2,6-difluorobenzyl)-1H-1,2,3-triazolo[4,5-c]pyridine comprenant la réaction de la 4-amino-1-(2,6-difluorobenzyl)-1H-1,2,3-triazolo[4,5-c] pyridine avec l'acide chlorhydrique.

17. Procédé pour la production d'un composé de formule (II) 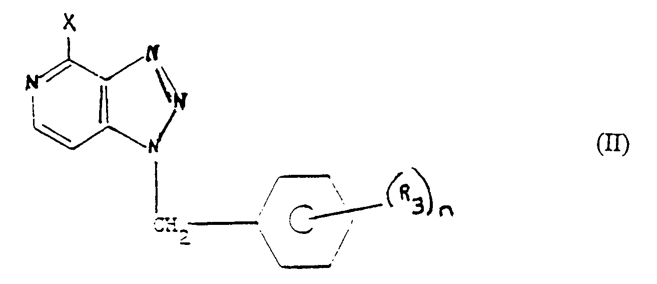 et des sels de celui-ci
dans laquelle X est un membre quelconque du groupe comprenant un halogène, un groupement alkylthio en C₁₋₆, arylthio en C₆₋₁₀, aralkylthio en C₇₋₁₂, alkyle en C₁₋₄, phényle, benzyle, phényléthyle, naphtylméthyle et sulfonyle ou sulfinyle substitué ;
R3 est un hydrogène ou un halogène et n est 1 ou 2,
ledit procédé comprenant la réaction d'un composé de formule (III) 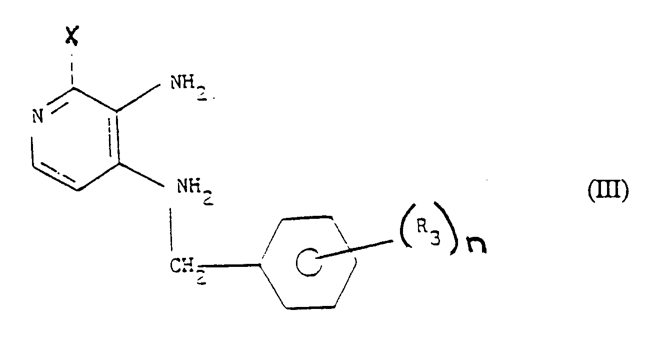 dans lequel X, R₃ et n sont tels que définis ci-dessus, avec du nitrite de sodium dans un acide hydrohalogéné.
